# EUROPEAN PATENT APPLICATION

(11) **EP 2 434 007 A1**
(43) Date of publication of application: **28.03.2012**
(21) Application number: 10777837.5
(22) Date of filing: 21.05.2010
(51) Int. Cl.: C12N 5/10, A61K 35/12, A61L 27/00, C12N 5/0735

(54) **METHOD FOR INDUCING DIFFERENTIATION OF EMBRYONIC STEM CELLS OR ARTIFICIAL PLURIPOTENT STEM CELLS**

(30) Priority: 22.05.2009 JP 2009141645
(71) Applicant: Tokyo Women's Medical University, Tokyo 162-8666 (JP)
(72) Inventor: SASAKI, Daisuke, Tokyo 162-8666 (JP); SHIMIZU, Tatsuya, Tokyo 162-8666 (JP); OKANO, Teruo, Tokyo 162-8666 (JP)
(74) Representative: HOFFMANN EITLE
(86) International application number: PCT/JP2010/058661
(87) International publication number: WO 2010/134606

(57) **Abstract**

Differentiation of a large number of embryonic stem cells or artificial pluripotent stem cells such as induced pluripotent stem cells can be readily induced by the following method. A substrate surface having a cell non-adhesive region on the periphery of a cell adhesive region is used, and
(1) the cells are allowed to adhere to the cell adhesive region of the substrate surface in a differentiation-inducing medium;
(2) the cells are subsequently allowed to grow on the cell adhesive surface to reach confluence;
(3) the culture is further continued to three-dimensionally stratify the cells from the boundary with the cell non-arlhesive region surrounding the cell adhesive region; and
(4) a plurality of the stratified portions formed in the cell adhesive region is finally bound to one another to produce a cell agglomerate spreading over the entire cell adhesive region with a certain thickness.

## Description

### TECHNICAL FIELD

The present invention relates to a method for inducing differentiation of embryonic stem cells or artificial pluripotent stem cells, for example, induced pluripotent stem cells, useful in the fields of, for example, drug discovery, pharmaceutics, medicine, and biology.

### BACKGROUND ART

In recent years, regenerative therapy based on cell transplantation has received attention as a therapeutic technology for replacing organ transplantation. Embryonic stem cells (ES cells), which have pluripotency and reproductive integrity, are expected as a cell source for preparing cells necessary for regenerative therapy. Furthermore, in recent years, it has been shown that induced pluripotent stem cells (iPS cells) having properties corresponding to ES cells can be prepared from adult somatic cells, and an expectation for clinical application of these cells has been increasingly high.

In order to achieve clinical application of ES cells, reproducible control of differentiation of the ES cells is indispensable. It has been revealed that formation of an aggregate of ES cells induces multicellular interaction in the aggregate to accelerate differentiation of the ES cells to various cells of triploblasts, such as myocardial cells, hepatocytes, and nerve cells. The ES cell aggregate is generally prepared as a spherical aggregate, called embryoid body, in a floating state. The differentiation of ES cells depends on the size of the embryoid body. Accordingly, it is necessary to control the size of the embryoid body for efficiently inducing differentiation into specific cells.

Under such circumstances, Non-Patent Document 1 describes a hanging drop method, which is a known method for controlling the sizes of embryoid bodies. Specifically, a cell aggregate, called embryoid body (EB), is formed by hanging droplets containing several hundred to several thousand ES cells from a lid of a cell culture dish in a medium and culturing the cells for 2 days. The cells are subjected to suspension culture in a cell non-adherent culture dish for several days and are then seeded on a cell adherent culture dish, followed by culturing for several days. The developed cell group of the EB adhering to the culture dish is called EB outgrowth. Beating myocardial cells are observed in 5% to 50% of EB outgrowth by culturing mouse ES cells under conditions inducing differentiation through the hanging drop method (Non-Patent Documents 2, 4, and 5). On this occasion, the rate of the number of differentiated myocardial cells to the total number of cells usually ranges from 1% to 3% (Non-Patent Document 6). Unfortunately, the hanging drop method requires a complicated procedure and therefore has a disadvantage of difficulty in preparation of a large number of embryoid bodies. In order to further differentiate ES cells in the embryoid body form, it is necessary to let the embryoid bodies in a floating state adhere onto a culture substrate and to subsequently culture them in many cases.

In addition, chemical substances that accelerate induction of differentiation into myocardial cells have been diligently being searched. Representative differentiation-inducing substances that have been found at present are, for example, retinoic acid in Non-Patent Document 2, ascorbic acid in Patent Document 1 and Non-Patent Document 3, nitrogen monoxide in Non-Patent Document 4, and nogin in Non-Patent Document 5. The rate of inducing differentiation into myocardial cells can be enhanced by adding these substances to media at appropriate concentrations and timing. Furthermore, various methods for enhancing induction of differentiation into myocardial cells have been investigated, for example, Patent Document 2 describes a method of culturing in a serum-free medium and Patent Document 3 describes a method of coculturing with cells that excrete a factor for inducing differentiation into myocardial cells. However, these methods still involve a risk that the cells not differentiated into objective cells cause trouble after transplantation; thus there is a demand for a culture technology enabling ready preparation of a sufficient number of cells necessary for transplantation.

### CITATION LIST

### PATENT DOCUMENT

Patent Document 1: PCT Japanese Translation Patent Publication No. 2008-523823
Patent Document 2: PCT Japanese Translation Patent Publication No. 2008-500821
Patent Document 3: PCT Japanese Translation Patent Publication No. 2006-523091

### NON-PATENT DOCUMENT

Non-Patent Document 1: Circ. Res., 2002, 91(3): 189-201
Non-Patent Document 2: J. Mol. Cell Cardiol., 1997, 29(6): 1525-39
Non-Patent Document 3: Circulation, 2003, 107(14): 1912-6
Non-Patent Document 4: Proc. Natl. Acad. Sci. USA., 2004, 101(33): 12277-81
Non-Patent Document 5: Nat. Biotechnol., 2005, 23(5): 607-11
Non-Patent Document 6: Cell, 2008, 132(4): 661-80

### SUMMARY OF INVENTION

### TECHNICAL PROBLEM

The present invention has been made to solve the problems involved in the above-described methods of inducing differentiation of embryonic stem cells or artificial pluripotent stem cells such as induced pluripotent stem cells. That is, the present invention provides a novel method of inducing differentiation of, for example, embryonic stem cells, on the basis of an absolutely different idea from conventional Technologies.

### SOLUTION TO PROBLEM

In order to solve the above-mentioned problems, the present inventors have conducted research and development through investigating from various angles and, as a result, have found that differentiation of embryonic stem cells or artificial pluripotent stem cells such as induced pluripotent stem cells can be induced by allowing the cells to adhere to a culture substrate and to form a specific three-dimensional structure and have further found that the differentiation induction can provide myocardial cells. The present invention has been completed based on these findings.

That is, the present invention provides a method of inducing differentiation of embryonic stem cells or artificial pluripotent stem cells such as induced pluripotent stem cells by allowing the embryonic stem cells or the artificial pluripotent stem cells to adhere to a culture substrate and to form a specific three-dimensional structure. The present invention also provides a method of inducing differentiation of embryonic stem cells or artificial pluripotent stem cells such as induced pluripotent stem cells into myocardial cells. In addition, the present invention provides differentiated stem cells obtained by such a method of inducing differentiation and also a method of transplantation therapy using the stem cells.

### ADVANTAGEOUS EFFECTS OF INVENTION

In the method of differentiation induction according to the present invention, embryonic stem cells or artificial pluripotent stem cells such as induced pluripotent stem cells can be easily induced into objective cells by a simple technique. Such a technique conventionally needs labors and skills by workers, but the present invention does not need them and can treat a large number of cells.

### BRIEF DESCRIPTION OF DRAWINGS

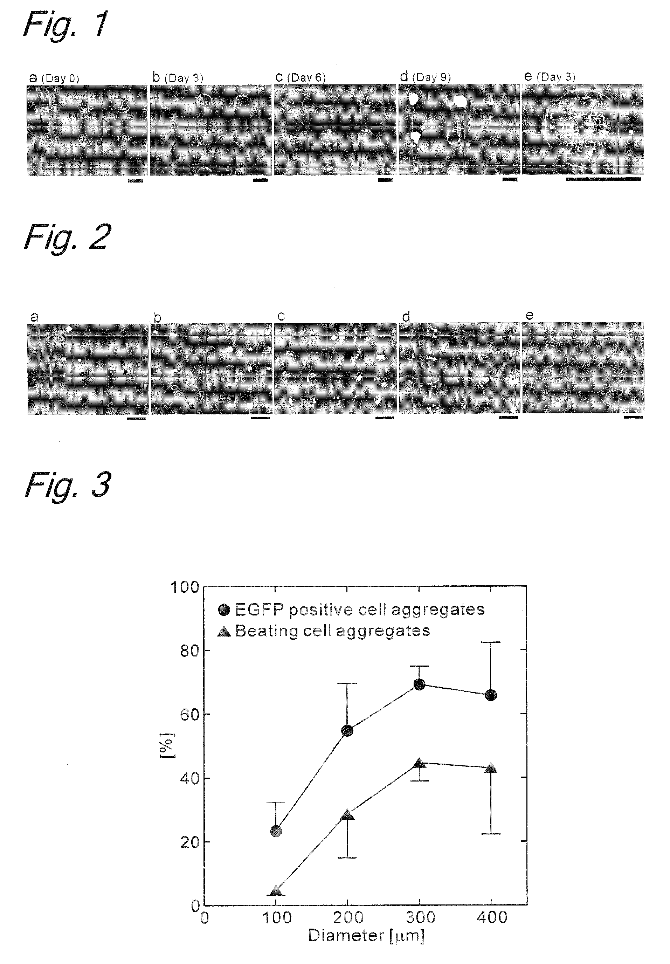

Fig. 1 shows the results of formation of ES cell aggregates and induction of differentiation into myocardial cells on cell-patterning culture substrates of Example 1: microscopic images of ES cells subjected to differentiation-inducing culture for 3 hr after seeding (a), 3 days (b and e), 6 days (c), or 9 days (d) on the substrates including circular cell adhesive regions each having a diameter of 200 µm. The saturated white portions show fluorescence of EGFP. The scale bars each represent 200 µm.

Fig. 2 shows the results of formation of ES cell aggregates and induction of differentiation into myocardial cells on cell-patterning culture substrates of Example 1: microscopic images of ES cells subjected to differentiation-inducing culture for 10 days on the substrates including circular cell adhesive regions each having a diameter of 100 µm (a), 200 µm (b), 300 µm (c), or 400 µm (d) or on a silanated cover glass surface (e) to which polyacrylamide is not fixed. The saturated white portions show fluorescence of EGFP. The scale bars each represent 500 µm.

Fig. 3 shows the results of formation of ES cell aggregates and induction of differentiation into myocardial cells on cell-patterning culture substrates of Example 1: a graph showing relationships between the diameter of the circular cell adhesive regions and the rate of cell aggregates (circular plots) exhibiting EGFP fluorescence or the rate of cell aggregates (triangular plots) exhibiting beating after differentiation-inducing culture for 10 days. One hundred cell aggregates were analyzed for each experiment. The error bars each represent the standard deviation of five independent experiments.

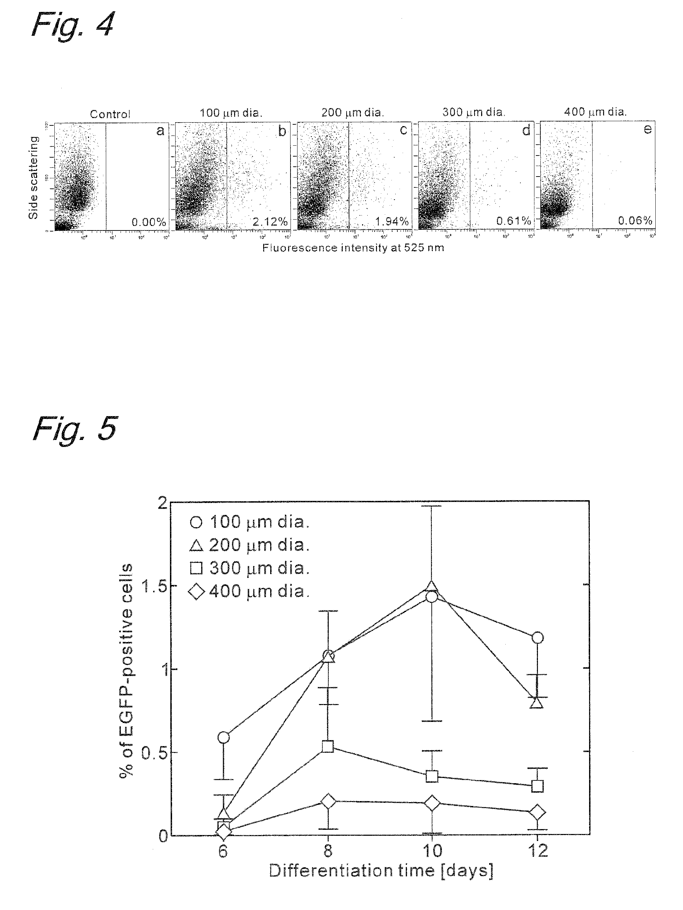

Fig. 4 shows the results of induction efficiency of differentiation into myocardial cells in Example 1: typical cellular distribution in flow cytometry of ES cells subjected to differentiation-inducing culture for 10 days on a silanated cover glass surface (a) to which polyacrylamide is not fixed or on a substrate including circular cell adhesive regions each having a diameter of 100 µm (b), 200 µm (c), 300 µm (d), or 400 µm (e). Fifty thousand cells were analyzed for each experiment.

Fig. 5 shows the results of induction efficiency of differentiation into myocardial cells in Example 1: time series of EGFP-positive cell ratios in ES cells subjected to differentiation-inducing culture on a substrate including circular cell adhesive regions each having a diameter of 100 µm (circular plots), 200 µm (triangular plots), 300 µm (quadrate plots), or 400 µm (rhomboidal plots), analyzed by flow cytometry. Fifty thousand cells were analyzed for each experiment. The error bars each represent the standard deviation of five independent experiments.

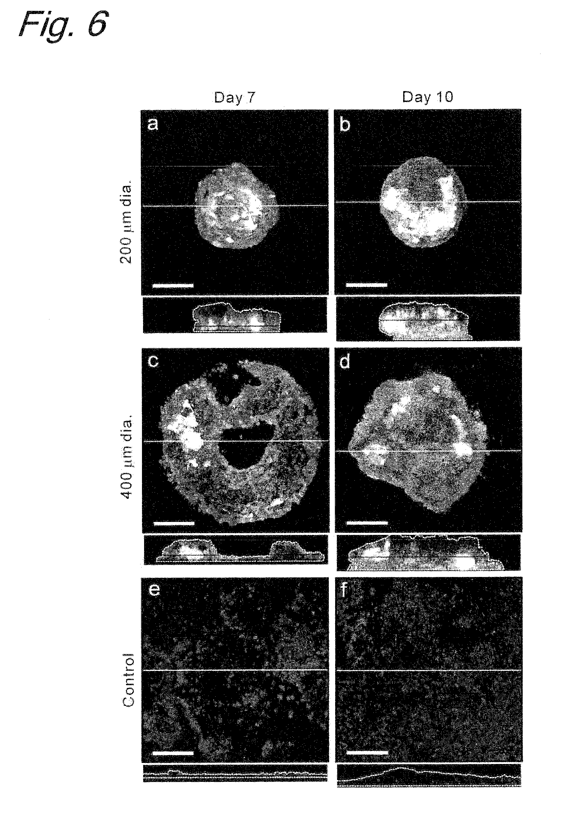

Fig. 6 shows the results of morphological analysis of differentiated ES cell aggregates in Example 1: confocal microscopic images of an ES cell aggregate formed by differentiation-inducing culture for 7 days (a) or 10 days (b) on a circular cell adhesive region having a diameter of 200 µm, an ES cell aggregate formed by differentiation-inducing culture for 7 days (c) or 10 days (d) on a circular cell adhesive region having a diameter of 400 µm, and an ES cells subjected to differentiation-inducing culture for 7 days (e) or 10 days (f) on a silanated cover glass surface to which polyacrylamide is not fixed. Each image on the upper side is a tomographical image of a section parallel to the culture substrate, and each image on the lower side is a tomographical image of a section perpendicular to the culture substrate. In the tomographical images, the outlines of the cell agglomerates are shown by dotted lines. The saturated white portions show fluorescence of EGFP. The scale bars each represent 100 µm.

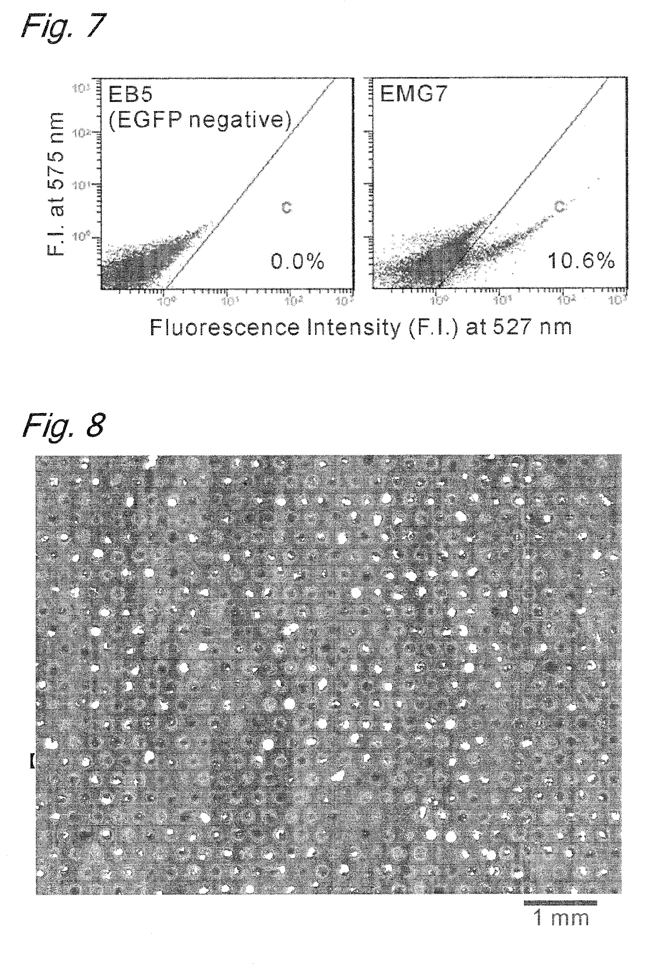

Fig. 7 shows the results of induction efficiency of differentiation into myocardial cells in Example 2: typical cellular distribution graphs in flow cytometry, showing induction efficiency of differentiation into myocardial cells of a mouse ES cell line EMG7 subjected to differentiation-inducing culture for 12 days. As an EGFP-negative control, a mouse ES cell line EB5 was subjected to differentiation-inducing culture under the same conditions.

Fig. 8 shows the results of formation of ES cell aggregates and induction of differentiation into myocardial cells on a cell-patterning culture substrate of Example 3: a microscopic image of ES cells subjected to differentiation-inducing culture for 12 days on a substrate including circular cell adhesive regions each having a diameter of 200 µm. The saturated white portions show fluorescence of EGFP. The scale bar represents 1 mm.

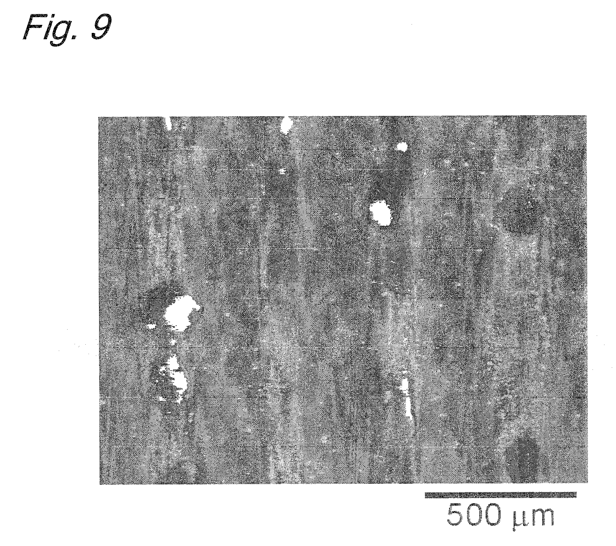

Fig. 9 shows the results of formation of ES cell aggregates and induction of differentiation into myocardial cells on a cell-patterning culture substrate of Example 4: a microscopic image of ES cells subjected to differentiation-inducing culture for 10 days on a substrate having a linear cell adhesive region with a width of 100 µm. The saturated white portions show fluorescence of EGFP. The scale bar represents 500 µm.

### DESCRIPTION OF EMBODIMENTS

The present invention relates to a method for inducing differentiation of embryonic stem cells or artificial pluripotent stem cells such as induced pluripotent stem cells using a substrate surface having a cell non-adhesive region on the periphery of a cell adhesive region. The material of the cell culture substrate provided with a coating may be made of not only a material usually used for cell culture, such as glass, modified glass, polystyrene, or polymethyl methacrylate, but also a material that can be generally formed into any shape, such as a polymer other than those mentioned above, a ceramic, or a metal. The shape is not limited to those of cell culture dishes such as petri dishes and may be a plate, a fiber, or a (porous) particle. Furthermore, the substrate may have a container-like shape (such as a flask) that is generally used for cell culture and other processes.

In the present invention, the cell adhesive region and the cell non-adhesive region are formed on a surface of the cell culture substrate. The cell adhesive region is a substrate surface to which cells can adhere, and the material thereof may be not only a material used as an ordinary culture substrate as described above, such as glass, modified glass, polystyrene, or polymethyl methacrylate, but also a material that can be generally formed into any shape, such as a polymer other than those mentioned above, a ceramic, or a metal. Furthermore, these materials may be subjected to surface treatment with, for example, glow discharge or corona discharge or may be coated with one or more of cell-adhesive proteins such as fibronectin, laminin, or collagen. The cell non-adhesive region used in the present invention is a region to which cells do not adhere and can be obtained by coating a material that prevents cells from adhering onto the above-mentioned culture substrate. Examples of the material suitable for achieving this purpose include, but not limited to, polyacrylamide, polyethylene glycol, starch, and mixtures thereof.

Examples of the shapes of the cell adhesive region and the surrounding cell non-adhesive region viewed from above include, but not limited to, (1) patterns composed of lines and spaces, (2) patterns of dots, (3) grid patterns, patterns having other specific shapes, and patterns of combination thereof. Though the size of the coated region is not particularly limited in the present invention, embryonic stem cells or artificial pluripotent stem cells such as induced pluripotent stem cells adhering to the substrate grow confluent in the cell adhesive region and become over confluent by continuing the culture to start stratification from the vicinity of the boundary between the cell adhesive region and the cell non-adhesive region, as described below. The mechanism can be explained by the fact that contact inhibition does not occur in the cells at the boundary between the cell adhesive region and the cell non-adhesive region even after the cells become confluent in the cell adhesive region and the cell growth is therefore not inhibited. In the present invention, in order to obtain high differentiation efficiency into objective cells, a plurality of stratified portions formed in the cell adhesive region need to rapidly bind to each other. Therefore, the cell adhesive region and the surrounding cell non-adhesive region are required to have shapes to achieve the binding at least in a part of the cell adhesive region. Accordingly, the distance between at least two stratified portions that are formed from the boundary to the surrounding cell non-adhesive region and bind to each other in the cell adhesive region is preferably 300 µm or less, more preferably 250 µm, and most preferably 200 µm or less. If the distance between two stratified portions that to be bound to each other is greater than 300 µm, disadvantageously, the binding of the stratified portions does not occur within a predetermined culturing time. The stratified portions may be stacked in any form: adjacent stratifying portions formed in the cell adhesive region may be stacked; in the case of a cell adhesive region having a circular shape, stratifying portions formed at both ends across the diameter may be stacked; or in the case of a cell adhesive region having a linear shape, stratifying portions formed on both ends across the width of the line may be stacked.

The cell adhesive region and the surrounding cell non-adhesive region of the present invention may be produced by any method that can finally provide the above-described structure. Examples of the method include (1) a method that involves masking of a surface of a cell adhesive substrate in the area for the cell adhesive region and coating the surface with the above-mentioned polymer only in the area for the cell non-adhesive region, (2) a method that involves off-set print of a cell non-adhesive polymer on a surface of a cell adhesive substrate, (3) a method that involves coating of two layers of a cell adhesive polymer and a cell non-adhesive polymer on a substrate in advance, and removal of either one of the layers with ultrasonic waves or a scanning machine (in this case, the cell adhesive polymer and the cell non-adhesive polymer may coat a culture substrate in this order, or inversely, the cell non-adhesive polymer and the cell adhesive polymer may coat a culture substrate in this order), (4) a method that involves production of a cell non-adhesive region on the entire surface of a substrate and spraying a material that finally constitutes the cell adhesive region thereon, or a method in an inverted manner. These methods may be employed alone or in combination.

The coating of the cell culture substrate with each polymer may be performed through a method (1) of binding the substrate and the polymer by a chemical reaction and/or a method (2) utilizing a physical interaction between the substrate and the polymer. That is, the method (1) of binding by a chemical reaction can employ, for example, electron beam (EB) irradiation, γ-raw irradiation, ultraviolet-ray irradiation, plasma treatment, or corona treatment. For a supporting material and a coating material having appropriate reactive functional groups, any common organic reaction, such as a radical reaction, an anionic reaction, or a cationic reaction, can be employed. In the method (2) of physical interaction, a coating material may be used alone or a matrix compatible with a supporting material may be used as a medium (for example, a graft polymer or a block polymer of a monomer forming the supporting material or a monomer compatible with the supporting material and the coating material) for physical adsorption, such as application or kneading.

In the present invention, the cell adhesive region of a cell culture substrate may be coated with a temperature-responsive polymer. In such a case, the temperature-responsive polymer preferably has an upper or lower critical solution temperature of 0°C to 80°C, more preferably 20°C to 50°C, in an aqueous solution. An upper or lower critical solution temperature higher than 80°C may cause undesirable death of cells. An upper or lower critical solution temperature lower than 0°C usually causes a significant reduction in cell growth rate or undesirable death of cells. The temperature-responsive polymer that is used in the present invention may be a homopolymer or a copolymer. Examples of these polymers are described in, for example, Japanese Unexamined Patent Application Publication No. Hei 2-211865. Specifically, the temperature-responsive polymer can be prepared by, for example, homopolymerization or copolymerization of the following monomers. Examples of the usable monomer include (meth)acrylamide compounds, N-(or N,N-di)alkylsubstituted (meth)acrylamide derivatives, and vinyl ether derivatives. In the case of copolymers, any two or more of these monomers can be used. Furthermore, the monomers may be copolymerized with other monomers, or the resulting polymers may be subjected to graft polymerization or copolymerization. Mixtures of these polymers and copolymers may also be used. The polymers can also be crosslinked to the extent that will not impair their inherent properties. Since cells are cultured and detached, separation is performed in a temperature range of 5°C to 50°C. Accordingly, examples of the temperature-responsive polymer include poly-N-n-propylacrylamide (lower critical solution temperature of homopolymer: 21°C), poly-N-n-propylmcthacrylamide (lower critical solution temperature of homopolymer: 27°C), poly-N-isopropylacrylamide (lower critical solution temperature of homopolymer: 32°C), poly-N-isopropylmethacrylamide (lower critical solution temperature of homopolymer: 43°C), poly-N-cyclopropylacrylamide (lower critical solution temperature of homopolymer: 45°C), poly-N-ethoxyethylacrylamide (lower critical solution temperature of homopolymer: about 35°C), poly-N-ethoxyethylmethacrylamide (lower critical solution temperature of homopolymer: about 45°C), poly-N-tetrahydrofurfurylacrylamide (lower critical solution temperature of homopolymer: about 28°C), poly-N-tetrahydrofurfurylmethacrylamide (lower critical solution temperature of homopolymer: about 35°C), poly-N,N-ethylmethylacrylamide (lower critical solution temperature of homopolymer: 56°C), and poly-N,N-diethylacrylamide (lower critical solution temperature of homopolymer: 32°C). Nonlimiting examples of the monomer for copolymerization used in the present invention include polyacrylamide, poly-N,N-diethylacrylamide, poly-N,N-dimethylacrylamide, polyethylene oxide, polyacrylic acid and salts thereof, and aqueous polymers such as polyhydroxyethyl methacrylate, polyhydroxyethyl acrylate, polyvinyl alcohol, polyvinyl pyrrolidone, cellulose, and carboxymethyl cellulose.

The coating density of the temperature-responsive polymer on the cell adhesive region is desirably in the range of 1.1 to 2.3 µg/cm², preferably 1.4 to 1.9 µg/cm², and more preferably 1.5 to 1.8 µg/cm². A coating density of less than 1.1 µg/cm² precludes detachment of the cells from the polymer even if a stimulus is applied, which causes a significant reduction in work efficiency. In contrast, a coating density of 2.3 µg/cm² or more prevents the adhesion of cells to such a region, resulting in difficulty in insufficient adhesion.

The present invention relates to a method for inducing differentiation of embryonic stem cells or artificial pluripotent stem cells such as induced pluripotent stem cells by allowing these cells to adhere to a surface of the substrate mentioned above. The cells to be used may be any embryonic stem cell or artificial pluripotent stem cell, which are available from any supplier or through any production process. The cells of the present invention are not limited, and examples thereof include animal, insect, and plant cells and bacteria. Examples of the animal from which cells are derived include, but not limited to, human, monkey, dog, cat, rabbit, rat, nude mouse, mouse, guinea pig, hog, sheep, Chinese hamster, cattle, marmoset, and African green monkey.

The method for inducing differentiation of embryonic stem cells or artificial pluripotent stem cells such as induced pluripotent stem cells according to the present invention is as follows: The embryonic stem cells or artificial pluripotent stem cells before seeding are maintained in the undifferentiated state with a medium not inducing differentiation. The medium is changed to a differentiation-inducing medium about the time of seeding onto the above-described substrate surface, and the cells are seeded to the substrate surface of the present invention and are allowed to grow confluent in the cell adhesive region. In the confluent state, the culture is further continued with the differentiation-inducing medium to allow the cells to three-dimensionally stratify from the boundary with the cell non-adhesive region surrounding the cell adhesive region. The culture is continued to finally obtain a cell agglomerate spreading over the entire cell adhesive region with a certain thickness by binding a plurality of stratified portions formed in the cell adhesive region to one another. The resulting cell agglomerate is treated with an enzyme to obtain objective differentiated cells.

In the present invention, any medium that does not induce differentiation of embryonic stem cells or artificial pluripotent stem cells such as induced pluripotent stem cells can be used as the medium not inducing differentiation, and examples of such a medium include media containing a leukemia inhibitory factor having a property of maintaining undifferentiation of mouse embryonic stem cells and mouse artificial pluripotent stem cells and media containing a basic FGF having a property of maintaining undifferentiation of human embryonic stem cells and human artificial pluripotent stem cells. Similarly, any medium that induces differentiation of embryonic stem cells or artificial pluripotent stem cells can be used as the differentiation-inducing medium, and examples of media include serum-containing media and serum-free media containing known components as replacement for serum. The differentiation-inducing medium may further contain a differentiation-inducing substance, such as retinoic acid or ascorbic acid, as mentioned above. The seeding density onto the substrate surface may be those in common methods and is not particularly limited. In the present invention, the density is adjusted such that the seeded cells become confluent in the cell adhesive region within typically eight days, preferably within seven days, and more preferably within six days. If it takes nine days or more for becoming confluent, the subsequent stratification cannot be sufficiently achieved, undesirably as the technology shown in the present invention. Accordingly, in the present invention, the area of the cell adhesive region on which cells are seeded and the number of cells to be seeded are adjusted such that the number of days from the seeding to becoming confluent is eight days or less.

The present invention is **characterized in that** culture of the embryonic stem cells or the artificial pluripotent stem cells is continued after the cells become confluent in the cell adhesive region. That is, since contact inhibition does not occur in the cells at the boundary between the cell adhesive region and the cell non-adhesive region even after the cells become confluent, in the cell adhesive region and the cell growth is therefore not inhibited, the cells grow towards the cell non-adhesive region surrounding the cell adhesive region. However, the cells cannot sufficiently adhere to the cell non-adhesive region. As a result, the cells grow and enlarge the culturing area in an insufficient state that the cells bind to the cells on the cell adhesive region, but do not sufficiently adhere to the surface of the substrate.
Finally, the cultured cells unstably adhering to the substrate surface are folded towards the cell adhesive region side from the vicinity of the boundary between the cell adhesive region and the cell non-adhesive region, are formed into a sheet-like shape to be folded towards the cell adhesive region side from the vicinity of the boundary between the cell adhesive region and the cell non-adhesive region, or rise to the cell adhesive region side from the vicinity of the boundary between the cell adhesive region and the cell non-adhesive region. At the same time, the cells having nowhere to go are piled up in the vicinity of the boundary. In the present invention, a string of these cells is folded and thereby the embryonic stem cells or the artificial pluripotent stem cells are placed under specific environmental conditions. As a result, differentiation of these stem cells is probably induced. The cells may be folded in any shape, but cells folded into a sheet form a space that is advantageous for efficiently promoting the subsequent induction of differentiation. Furthermore, it was found that the folded portion is differentiated into myocardial cells in the present invention. Observation of a cross-section of the resulting cell agglomerate revealed that the density of the cells is high at the outermost layer and that the density of the cells inside the agglomerate is low. The myocardial cells of the present invention are present inside the agglomerate in a larger number. It was suggested that there is a difference in the differentiation direction of cells between the outer layer and the inside of the agglomerate.

In the case where a temperature-responsive polymer is coated on the cell adhesive region of the present invention, the differentiated cells can be detached by adjusting the temperature of the culture substrate to a temperature not lower than the upper critical solution temperature or not higher than the lower critical solution temperature of the coating polymer on the culture substrate, with no enzyme treatment. The detachment can be performed in a culture solution or in another isotonic solution, and the solution can be selected depending on the purpose. In order to detach and harvest the cells more quickly and more efficiently, a method of lightly tapping or shaking the substrate and a method of stirring the medium with a pipette may be employed alone or in combination.

In the method of differentiation induction according to the present invention, embryonic stem cells or artificial pluripotent stem cells such as induced pluripotent stem cells can be easily induced into objective cells by a simple technique. Although conventional techniques need labors and skills by workers, the present invention does not need them and can treat a large number of cells. Furthermore, in the present invention, differentiation of a large number of embryonic stem cells or artificial pluripotent stem cells can be easily induced, while high induction efficiency of differentiation of the embryonic stem cells or the artificial pluripotent stem cells into specific cells is achieved, by designing the patterns of a cell adhesive region and a cell non-adhesive region on a surface of a culture substrate.

### EXAMPLES

The present invention will now be described in more detail with reference to examples below, which are not intended to limit the present invention.

### EXAMPLE 1

### Reagents

Reagents used in Example 1 are as follows.
3-Methacryloxypropyltrimethoxysilane (MPTMS) was purchased from Shin-Etsu Chemical Co., Ltd. A g-line positive photoresist (OFPR-800LB, 34cP) and a developing solution (NMD-3) were purchased from Tokyo Ohka Kogyo Co., Ltd. Acrylamide, N,N'-methylenebisacrylamide, ammonium persulfate, sodium hydrogen carbonate, sodium chloride, potassium chloride, magnesium sulfate, sodium pyruvate, taurine, sodium hydroxide, calcium chloride, dipotassium hydrogen phosphate, creatine, potassium hydroxide, and paraformaldehyde were purchased from Wako Pure Chemical Industries, Ltd. N,N,N',N'-Tetramethylethylenediamine (TEMED) and glucose were purchased from Kanto Chemical Co., Inc. D-PBS, Minimum Essential Medium Eagle Alpha Modification (α-MEM, Product Number: M0644), HEPES, and Na₂ATP were purchased from Sigma-Aldrich Japan K.K. Fibronectin was purchased from Biomedical Technologies Inc. Fetal calf serum was purchased from Nichirei Biosciences Inc. 2-Mercaptoethanol, penicillin-streptomycin, TrypLE^{™} Express, CellTracker^{™} Orange CMTMR, and SlowFade Gold antifade reagent were purchased from Invitrogen Corp. EDTA and EGTA were purchased from Dojindo Molecular Technologies, Inc. Collagenase/dispase was purchased from Roche Diagnostics K.K.

### Production of cell patterning culture substrate

Culture substrates each having a cell culture surface provided with a pattern of cell adhesive regions and cell non-adhesive regions were produced based on the document (Itoga K, Kobayashi J, Yamato M, Kikuchi A, Okano T., Maskless liquid-crystal-display projection photolithography for improved design flexibility of cellular micropatterns, Biomaterials, 2006, 27(15): 3005-9). A cover glass (24 mm × 50 mm, thickness: 0.12 to 0.17 mm, Matsunami Glass Ind., Ltd.) was treated with silane by an evaporation process using MPTMS. A g-line positive photoresist was spin-coated (5000 rpm, 30 sec) on the silane-treated cover glass and was prebaked at 80°C for 1 hr. A photoresist pattern of circles each having a diameter of 100, 200, 300, or 400 µm arrayed in a square at intervals of 300 µm was formed on the substrate surface by photolithography using a maskless exposure apparatus. The substrate was post-baked at 80°C for 1 hr. Dissolved oxygen in an aqueous solution containing 0.7 mM acrylamide and 65 µM N,N'-methylenebisacrylamide was removed by bubbling of nitrogen gas at room temperature for 20 min, and the solution was cooled to 4°C. After ammonium persulfate (2.2 mM) and TEMED (11 mM) were added to the solution, the photoresist-patterned substrate was immediately immersed in this aqueous solution. The aqueous solution was left to stand at 4°C for 3 hr with gentle stirring to fix polyacrylamide to the silanated glass surface of the substrate at the portion not coated with the photoresist. The substrate was sufficiently washed with warm water at about 40°C to remove the polyacrylamide that was not fixed to the silanated glass surface and was subsequently washed with acetone to remove the photoresist pattern of the circles on the substrate surface. The resulting substrate surface had cell adhesive circular regions made of the silanated glass surface and cell non-adhesive regions made of the polyacrylamide surface. The substrate was sufficiently washed with deionized water, dried, cut into a size of 24 mm × 20 mm, placed in a petri dish having a diameter of 35 mm, and sterilized with ethylene oxide gas. In order to enhance the adhesion of cells to the circular regions before seeding, the substrate was immersed in 2 mL of D-PBS containing 30 µg/mL of fibronectin at 37°C overnight for coating the substrate surface with fibronectin.

### Cell culture

The experiment used a mouse ES cell line EMG7 (Yamashita JK, Takano M, Hiraoka-Kanie M, Shimazu C, Peishi Y, Yanagi K, et al., Prospective identification of cardiac progenitors by a novel single cell-based cardiomyocyte induction, FASEB J., 2005, 19(11): 1534-6) having an introduced gene expressing EGFP under the control of a myocardical α-myosin heavy chain promoter. Undifferentiated ES cells were subcultured on a culture dish coated with gelatin in accordance with the culturing process described in the document. Differentiation was induced in a differentiation-inducing medium that was an α-MEM containing 5% fetal calf serum, 50 µM 2-mercaptoethanol, 5 unit/mL of penicillin, and 5 ug/mL of streptomycin. Undifferentiated ES cells collected by TrypLE^{™} Express treatment (37°C, 5 min) and suspended in the differentiation-inducing medium were seeded at 2 × 10⁴ cells/cm² on the culture substrate produced by the above-described process, and the ES cells were then cultured at 37°C in 5% carbon dioxide under saturated vapor pressure. The medium was replaced once at 3 hr after the seeding of the cells to remove the ES cells remaining on the cell non-adhesive region. Afterward, the medium was replaced once a day. As a control experiment, ES cells were cultured for differentiation induction under the same conditions on a silanated glass surface to which polyacrylamide was not fixed. The cultured cells were observed with an inverted microscope (Eclipse TE2000-U, Nikon Corporation) equipped with a HiSCA CCD camera (model C6790, Hamamatsu Photonics K.K.), and microscopic images were obtained with image analysis software AQUACOSMOS (Hamamatsu Photonics K.K.). EGFP fluorescence was observed using a dedicated optical filter (XF116-2, Omega Optical, Inc.).

### Formation of ES cell aggregates and induction of differentiation into myocardial cell on cell patterning culture substrate

The ES cells cultured for differentiation induction on the substrate having cell adhesive regions each having a diameter of 200 µm were subjected to time series observation with a microscope. Fig. 1 shows the microscopic images. The ES cells seeded on the substrate grew in the circular cell adhesive regions and reached confluence on the 3rd day of the culture to form circular cell colonies (Figs. 1b and 1e). As shown in the enlarged microscopic image (Fig. 1e), the edge of the circular cell colony was very clear to show that fine patterning of the cells was achieved. The ES cells at the boundary between the ell adhesive region and the cell non-adhesive region further grew after the cells became confluent, and these growing cells stratified on the cell adhesive region to gradually form three-dimensional ES cell aggregates (Figs. 1c and 1d). Many of the finally formed ES cell aggregates had a three-dimensional structure including an outermost layer having a high cell density and an inside having a low cell density. Around the 7th day of the culture, clear EGFP fluorescence indicating differentiation into myocardial cells was observed, and some of the cell aggregates showing EGFP fluorescence started beating. Fig. 2 shows microscopic images of ES cells subjected to differentiation-inducing culture for 10 days on the substrates including cell adhesive regions each having a diameter of 100 µm, 200 µm, 300 µm, or 400 µm. ES cell aggregates were formed on the circular cell adhesive regions of every substrate, and EGFP fluorescence indicating differentiation into myocardial cells and beating were confirmed. However, in the case of cell adhesive regions having a diameter of 100 µm, a half or more of formed cell aggregates were lost by detachment from the substrate during replacement of the medium (Fig. 2a). The ES cells cultured for differentiation induction as a control experiment under the same conditions on a silanated cover glass surface to which polyacrylamide was not fixed did not form a three-dimensional aggregate on the substrate (Fig. 2e), and EGFP fluorescence and beating were barely observed. The ratio of cell aggregates showing EGFP fluorescence and the ratio of cell aggregates showing beating among all the cell aggregates formed by culturing for differentiation induction for 10 days on the substrate were analyzed by microscopic observation (Fig. 3). Both ratios increased with an increase in the diameter of the cell adhesive region and reached a maximum at a diameter of 300 µm and a diameter of 400 µm. The ratio of the cell aggregates showing EGFP fluorescence reached up to about 70%, and the ratio of the cell aggregates showing beating reached up to about 45%.

### Flow cytometry

In order to perform flow cytometry of the differentiated ES cells, single cell suspension was prepared from the differentiated ES cell aggregates by a method where some modifications were applied to the method described in the document (Wobus AM, Guan K, Yang HT, Boheler KR, Embryonic stem cells as a model to study cardiac, skeletal muscle, and vascular smooth muscle cell differentiation, Methods Mol. Biol., 2002, 185: 127-56). The ES cells cultured for differentiation induction on the substrate were washed with a low-calcium ion medium (a solution composed of 120 mM sodium chloride, 5.4 mM potassium chloride, 5 mM magnesium sulfate, 10 mM HEPES, 5 mM sodium pyruvate, 20 mM glucose, and 20 mM taurine and having a pH of 6.9 adjusted with sodium hydroxide) and were immersed in the low-calcium ion medium containing 1 mg/mL of collagenase/dispase and 30 µM calcium chloride at 37°C for 40 min. The cells were collected in a centrifuge tube and were precipitated by centrifugation (100 × g, 5 min), and the supernatant was removed. Subsequently, the cells were suspended in D-PBS containing 10% TrypLE^{™} Express. 1 mM EDTA, and 20 mM taurine, followed by leaving to stand at 37°C for 30 min. The cells were precipitated by centrifugation (300 × g, 5 min), and the supernatant was removed carefully. Subsequently, the cells were suspended in a KB medium (a solution composed of 85 mM potassium chloride, 30 mM dipotassium hydrogen phosphate, 5 mM magnesium sulfate, 1 mM EGTA, 5 mM Na₂ATP, 5 mM sodium pyruvate, 5 mM creatine, 20 mM glucose, and 20 mM taurine and having a pH of 7.2 adjusted with potassium hydroxide), and the cells were separated into individual cells by applying shearing stress to the cluster of the cells through repeating suction and discharge of the cell suspension with a pipette. Furthermore, this cell suspension was filtered through a nylon mesh with an opening of 35 µm to remove remaining cluster of the cells, and the filtrate was subjected to flow cytometry with a flow cytometer EPICS XL (Beckman Coulter, Inc.).

### Induction efficiency of differentiation into myocardial cells

Single cell suspension was prepared from the ES cell aggregates cultured for differentiation induction and was analyzed for the numerical ratio of EGFP-positive cells to the total cells by flow cytometry (Fig. 4). In the control experiment, EGFP fluorescence was not substantially observed in the ES cells cultured for differentiation induction on the silanated cover glass surface to which polyacrylamide was not fixed. Accordingly, this was used as an EGFP-negative control (Fig. 4a). The ratio of EGFP-positive cells, that is, myocardial cells differentiated from ES cells, reached a maximum on the 10th day of the culture for differentiation induction in the cell adhesive region having a diameter of 100 µm or 200 µm and was 1.5 ± 0.5% (average ± standard deviation, the number of samples: 5) (Fig. 5). The ratio of EGFF-pasitive cells rather decreased from the 10th to the 12th days of the culture for differentiation induction. This was probably caused by growth of cells other than myocardial cells.

### Confocal microscopic observation

In order to fluorescently stain the cytoplasm of the ES cells cultured for differentiation induction on the substrate, the cells were cultured in an α-MEM containing 10 µM CellTracker^{™} Orange CMTMR for 1 hr at 37°C in 5% carbon dioxide under saturated vapor pressure and subsequently in a differentiation-inducing medium for 1 hr. The cells were fixed with D-PBS containing 2% formalin (room temperature, 15 min) and were gently washed with D-PBS. The cells were coated with an antifade reagent (SlowFade Gold antifade reagent) and were placed and sealed between slide glasses (76 × 26 mm, Matsunami Glass Ind., Ltd.) with a silicon spacer having a thickness of 1.5 mm along the edges of the substrate. The cells were observed with a confocal microscope LSM510 (Carl Zeiss AG).

### Morphological analysis of differentiated ES cell aggregate

The ES cell aggregates formed by culturing for differentiation induction on the cell adhesive regions having a diameter of 200 µm or 400 µm and the ES cells cultured for differentiation induction on the silanated cover glass surface to which polyacrylamide was not fixed in the control experiment were subjected to morphological analysis by observation under a confocal microscope (Fig. 6). In the ES cells cultured for differentiation induction on the cell adhesive region having a diameter of 200 µm, on the 7th day of the culture, a three-dimensional aggregate was already formed and EGFP fluorescence appeared (Fig. 6a). On the 10th day of the culture, the cell aggregate formed a three-dimensional structure including an outermost layer having a high cell density and an inside having a low cell density, and in the inside having a low cell density, EGFP expressing region was enlarged (Fig. 6b). In the ES cells cultured for differentiation induction on the cell adhesive region having a diameter of 400 µm, on the 7th day of the culture, a cell aggregate was formed only in the vicinity of the boundary between the cell adhesive region and the cell non-adhesive region (the area from the edge of the cell adhesive region to about 150 µm towards the inside) and EGFP fluorescence appeared inside the formed aggregate (Fig. 6c). On the 10th day of the culture, a three-dimensional cell aggregate was formed on the entire cell adhesive region having a diameter of 400 µm, but the EGFP expressing region was limited on the area from the edge of the cell adhesive region to about 120 µm towards the inside (Fig. 6d). In the control experiment, the ES cells cultured for differentiation induction formed a single-layer structure without forming a three-dimensional aggregate on the substrate on the 7th day of the culture (Fig. 6e) and formed a layer structure having a small thickness (about 20 µm) on the 10th day of the culture (Fig. 6f). However, no EGFP fluorescence was observed in these samples.

### Example 2

### Reagents

Reagents used in Example 2 are shown below.
3-Methacryloxypropyltrimethoxysilane (MPTMS) was purchased from Shin-Etsu Chemical Co., Ltd. A g-line positive photoresist (OFPR-800LB, 34cP) and a developing solution (NMD-3) were purchased from Tokyo Ohka Kogyo Co., Ltd. Acrylamide, N,N'-methylenebisacrylamide, ammonium persulfate, sodium hydrogen carbonate, sodium chloride, potassium chloride, magnesium sulfate, sodium pyruvate, taurine, sodium hydroxide, calcium chloride, dipotassium hydrogen phosphate, creatine, potassium hydroxide, and L-ascorbic acid phosphate trisodium salt were purchased from Wako Pure Chemical Industries, Ltd. N,N,N',N'-Tetramethylethylenediamine (TEMED) and glucose were purchased from Kanto Chemical Co., Inc. D-PBS, Minimum Essential Medium Eagle Alpha Modification (α-MEM, Product Number: M0644), HEPES, and Na₂ATP were purchased from Sigma-Aldrich Japan K.K. Fibronectin was purchased from Becton, Dickinson and Company. Fetal calf serum was purchased from Nichirei Biosciences Inc. 2-Mercaptoethanol, penicillin-streptomycin, TrypLE^{™} Express, and Trypsin-EDTA were purchased from Invitrogen Corp. EDTA and EGTA were purchased from Dojindo Molecular Technologies, Inc. Collagenase/dispase was purchased from Roche Diagnostics K.K. DNase was purchased from Worthington Biochemical Corporation.

### Production of cell patterning culture substrate

Culture substrates each having a cell culture surface provided with a pattern of cell adhesive regions and cell non-adhesive regions were produced based on the document (Itoga K, Kobayashi J, Yamato M, Kikuchi A, Okano T., Maskless liquid-crystal-display projection photolithography for improved design flexibility of cellular micropatterns, Biomaterials, 2006, 27(1.5): 3005-9). A cover glass (24 mm, × 50 mm, thickness: 0.12 to 0.17 mm, Matsunami Glass Ind., Ltd.) was treated with silane by an evaporation process using MPTMS. A g-line positive photoresist was spin-coated (3000 rpm, 30 sec) on the silane-treated cover glass and was prebaked at 80°C for 1 hr. A photoresist pattern where circles each having a diameter of 200 µm were arrayed in a regular triangle at intervals of 50 µm was formed on the substrate surface by photolithography. The substrate was post-baked at 80°C for 1 hr. Dissolved oxygen in an aqueous solution containing 0.7 mM acrylamide and 65 µM N,N'-methylenebisacrylamide was removed by bubbling of nitrogen gas at room temperature for 20 min, and the solution was cooled to 4°C. After ammonium persulfate (2.2 mM) and TEMED (11 mM) were added to the solution, the photoresist-patterned substrate was immediately immersed in this aqueous solution. The aqueous solution was left to stand at 4°C for 3 hr with gentle stirring to fix polyacrylamide to the silanated glass surface of the substrate at the portion not coated with the photoresist. The substrate was sufficiently washed with warm water at about 40°C to remove the polyacrylamide that was not fixed to the silanated glass surface and was subsequently washed with acetone to remove the photoresist pattern of the circles on the substrate surface. The resulting substrate surface had cell adhesive circular regions made of the silanated glass surface and cell non-adhesive regions made of the polyacrylamide surface. The substrate was sufficiently washed with deionized water, dried, cut into a size of 24 mm × 20 mm, placed in a petri dish having a diameter of 35 mm, and sterilized width ethylene oxide gas. In order to enhance the adhesion of cells to the circular regions before seeding, the substrate was immersed in 2 mL of D-PBS containing 1 µg/mL of fibronectin at room temperature for 2 hr or more for coating the substrate surface with fibronectin.

### Cell culture

The experiment used mouse ES cell line EB5 (Hirai H, Ogawa M, Suzuki N, Yamamoto M, Breier G, Mazda O, et al., Hemogenic and nonhemogenic endothelium can be distinguished by the activity of fetal liver kinase (Flk)-1 promoter/enhancer during mouse embryogenesis, Blood, 2003, 101(3): 886-93) and a mouse ES cell line EMG7 (Yamashita JK, Takano M, Hiraoka-Kanie M, Shimazu C, Peishi Y, Yanagi K, et al., Prospective identification of cardiac progenitors by a novel single cell-based cardiomyocyte induction, FASEB J., 2005, 19(11): 1534-6), which is EB5 having an introduced gene expressing EGFP under the control of a myocardical α-myosin heavy chain promoter. Undifferentiated ES cells were subcultured on a culture dish coated with gelatin in accordance with the culturing process described in the document. Differentiation was induced in a differentiation-inducing medium that was an α-MEM containing 20% fetal calf serum, 0.5 mM L-ascorbic acid phosphate trisodium salt, 50 µM 2-mercaptoethanol, 5 unit/mL of penicillin, and 5 µg/mL of streptomycin. Undifferentiated ES cells collected by Trypsin-EDTA treatment (0.5%, 37°C, 5 min) and suspended in the differentiation-inducing medium were seeded at 3 × 10⁴ cells/cm² on the culture substrate produced by the above-described process, and the ES cells were cultured at 37°C in 5% carbon dioxide under saturated vapor pressure. The medium was replaced once at 3 hr after the seeding of the cells to remove the ES cells remaining on the cell non-adhesive region. Afterward, the medium was replaced once a day until the 4th day and twice a day after the 4th day.

### Formation of ES cell aggregate and induction of differentiation into myocardial cell on cell patterning culture substrate

The ES cells seeded on the substrate grew in the circular cell adhesive regions and reached confluence on the 3rd day of the culture to form circular cell colonies. The ES cells at the boundary between the ell adhesive region and the cell non-adhesive region further grew after the cells became confluent, and these growing cells stratified on the cell adhesive region to gradually form three-dimensional ES cell aggregates. Around the 10the day of the culture, clear EGFP fluorescence indicating differentiation into myocardial cells was observed, and some of the cell aggregates showing EGFP fluorescence started beating.

### Flow cytometry

In order to perform flow cytometry of the differentiated ES cells, single cell suspension was prepared from the differentiated ES cell aggregates by a method where some modifications were applied to the method described in the document (Wobus AM, Guan K, Yang HT, Boheler KR, Embryonic stem cells as a model to study cardiac, skeletal muscle, and vascular smooth muscle cell differentiation, Methods Mol. Biol., 2002, 185: 127-563. The ES cells cultured for differentiation induction on the substrate were washed with a low-calcium ion medium (a solution composed of 120 mM sodium chloride, 5.4 mM) potassium chloride, 5 mM magnesium sulfate, 1.0 mM HEPES, 5 mM sodium pyruvate, 20 mM glucose, and 20 mM taurine and having a pH of 6.9 adjusted with sodium hydroxide) and were immersed in the low-calcium ion medium containing 1 mg/mL of collagenase/dispase and 30 µM calcium chloride at 37°C for 40 min. The cells were collected in a centrifuge tube and were precipitated by centrifugation (200 × g, 3 min), and the supernatant was removed. Subsequently, the cells were suspended in TrypLE^{™} Express, followed by leaving to stand at 37°C for 20 min. The cells were precipitated by centrifugation (200 × g, 5 min), and the supernatant was removed carefully. Subsequently, the cells were suspended in a KB medium (a solution composed of 85 mM potassium chloride, 30 mM dipotassium hydrogen phosphate, 5 mM magnesium sulfate, 1 mM EGTA, 5 mM Na₂ATP, 5 mM sodium pyruvate, 5 mM creatine, 20 mM glucose, and 20 mM taurine and having a pH of 7.2 adjusted with potassium hydroxide) containing 600 U/mL of DNase, and the cells were separated into individual cells by applying shearing stress to the cluster of the cells through repeating suction and discharge of the cell suspension with a pipette. Furthermore, this cell suspension was filtered through a nylon mesh with an opening of 35 µm to remove remaining cluster of the cells, and the filtrate was subjected to flow cytometry with a flow cytometer EPICS XL (Beckman Coulter, Inc.).

### Induction efficiency of differentiation into myocardial cells

Single cell suspension was prepared from the aggregates of a mouse ES cell line EMG7 cultured for differentiation induction for 12 days and was analyzed for the numerical ratio of EGFP-positive cells to the total cells by flow cytometry (Fig. 7). A mouse ES cell line EB5 cultured for differentiation induction under the same conditions was used as an EGFP-negative control. The ratio of ECFP-positivc cells, that is, myocardial cells differentiated from ES cells, was 8.9 ± 1.5% (average ± standard deviation, the number of samples: 3).

### EXAMPLE 3

### Reagents

Reagents used in Example 3 are as follows.
3-Methacryloxypropyltrimethoxysilane (MPTMS) was purchased from Shin-Etsu Chemical Co., Ltd. A g-line positive photoresist (OFPR-800LB, 34cP) and a developing solution (NMD-3) were purchased from Tokyo Ohka Kogyo Co., Ltd. Acrylamide, N,N'-methylenebisacrylamide, ammonium persulfate, sodium hydrogen carbonate, and L-ascorbic acid phosphate trisodium salt were purchased from Wako Pure Chemical Industries, Ltd. N,N,N',N'-Tetramethylethylenediamine (TEMED) and glucose were purchased from Kanto Chemical Co., Inc. D-PBS and Minimum Essential Medium Eagle Alpha Modification (α-MEM, Product Number: M0644) were purchased from Sigma-Aldrich Japan K.K. Fibronectin was purchased from Becton, Dickinson and Company. Fetal calf serum was purchased from Nichirei Biosciences Inc. 2-Mercaptoethanol, penicillin-streptomycin, and TrypLE^{™} Express were purchased from Invitrogen Corp.

### Production of cell patterning culture substrate

Culture substrates each having a cell culture surface provided with a pattern of cell adhesive regions and cell non-adhesive regions were produced based on the document (Itoga K, Kobayashi J, Yamato M, Kikuchi A, Okano T., Maskless liquid-crystal-display projection photolithography for improved design flexibility of cellular micropatterns, Biomaterials, 21306, 27(15): 3005-9). A cover glass (24 mm × 50 mm, thickness: 0.12 to 0.17 mm, Matsunami Glass Ind., Ltd.) was treated with silane by an evaporation process using MPTMS. A g-line positive photoresist was spin-coated (3000 rpm, 30 sec) on the silane-treated cover glass and was prebaked at 80°C for 1 hr. A photoresist pattern where circles each having a diameter of 200 µm were arrayed in a regular triangle at intervals of 100 µm was formed on the substrate surface by photolithography. The substrate was post-baked at 80°C for 1 hr. Dissolved oxygen in an aqueous solution containing 0.7 mM acrylamide and 65 µM N.N'-methylenebisacrylamide was removed by bubbling of nitrogen gas at room temperature for 20 min, and the solution was cooled to 4°C. After ammonium persulfate (2.2 mM) and TEMED (11 mM) were added to the solution, the photoresist-patterned substrate was immediately immersed in this aqueous solution. The aqueous solution was left to stand at 4°C for 3 hr with gentle stirring to fix polyacrylamide to the silanated glass surface of the substrate at the portion not coated with the photoresist. The substrate was sufficiently washed with warm water at about 40°C to remove the polyacrylamide that was not fixed to the silanated glass surface and was subsequently washed with acetone to remove the photoresist pattern of the circles on the substrate surface. The resulting substrate surface had cell adhesive circular regions made of the silanated glass surface and cell non-adhesive regions made of the polyacrylamide surface. The substrate was sufficiently washed with deionized water, dried, cut into a size of 24 mm × 24 mm, placed in a petri dish having a diameter of 35 mm, and sterilized with ethylene oxide gas. In order to enhance the adhesion of cells to the circular regions before seeding, the substrate was immersed in 2 mL of D-PBS containing 5 µg/mL of fibronectin at room temperature for 1 hr or more for coating the substrate surface with fibronectin.

### Cell culture

The experiment used a mouse ES cell line EMG7 (Yamashita JK, Takano M, Hiraoka-Kanie M, Shimazu C, Peishi Y, Yanagi K. et al., Prospective identification of cardiac progenitors by a novel single cell-based cardiomyocyte induction, FASEB J., 2005, 19(11): 1534-6) having an introduced gene expressing EGFP under the control of a myocardical α-myosin heavy chain promoter. Undifferentiated ES cells were subcultured on the culture dish coated with gelatin in accordance with the culturing process described in the document (Hirai H, Ogawa M, Suzuki N, Yamamoto M, Breier G, Mazda O, et al., Hemogenic and nonhemogenic endothelium can be distinguished by the activity of fetal liver kinase (Flk)-1 promoter/enhancer during mouse embryogenesis, Blood, 2003, 101(3): 886-93). Differentiation was induced in a differentiation-inducing medium that was an α-MEM containing 20% or 10% fetal calf serum, 0.5 mM L-ascorbic acid phosphate trisodium salt, 50 µM 2-mercaptoethanol, 5 unit/mL of penicillin, and 5 µg/mL of streptomycin. Undifferentiated ES cells collected by TrypLE^{™} Express treatment (37°C, 7 min) and suspended in the differentiation-inducing medium were seeded at 3 × 10⁴ cells/cm² on the culture substrate produced by the above-described process, and the ES cells were then cultured at 37°C in 5% carbon dioxide under saturated vapor pressure. The medium was replaced once a day until the 4th day of the culture and twice a day after the 4th day. The differentiation-inducing medium used until the 6th day of the culture contained 20% fetal calf serum, and after the 6th day, a differentiation-inducing medium containing 10% fetal calf serum was used. The cultured cells were observed with an inverted microscope (Eclipse TE2000-U, Nikon Corporation) equipped with a HiSCA CCD camera (model C6790, Hamamatsu Photonics K.K.), and microscopic images were obtained with image analysis software AQUACOSMOS (Hamamatsu Photonics K.K.). EGFP fluorescence was observed using a dedicated optical filter (XF116-2, Omega Optical, Inc.).

### Formation of ES cell aggregate and induction of differentiation into myocardial cell on cell patterning culture substrate

The ES cells seeded on the substrate grew in the circular cell adhesive regions and reached confluence on the 3rd day of the culture to form circular cell colonies. The ES cells at the boundary between the ell adhesive region and the cell non-adhesive region further grew after the cells became confluent, and these growing cells stratified on the cell adhesive region to gradually form three-dimensional ES cell aggregates. Around the 10th day of the culture, clear EGFP fluorescence indicating differentiation into myocardial cells was observed, and some of the cell aggregates showing EGFP fluorescence started beating. Fig. 8 shows a microscopic image of cell aggregates formed on the substrate by culturing for differentiation induction for 12 days. The ratio of the cell aggregates showing EGFP fluorescence was about 60%.

### EXAMPLE 4

### Reagents

Reagents used in Example 4 are as follows. 3-Methacryloxypropyltrimethoxysilane (MPTMS) was purchased from Shin-Etsu Chemical Co., Ltd. A g-line positive photoresist (OFPR-800LB, 34cP) and a developing solution (NMD-3) were purchased from Tokyo Ohka Kogyo Co., Ltd. Acrylamide, N,N'-methylenebisacrylamide, ammonium persulfate, and sodium hydrogen carbonate were purchased from Wako Pure Chemical Industries, Ltd. N,N,N',N'-Tetramethylethylenediamine (TEMED) and glucose were purchased from Kanto Chemical Co., Inc. D-PBS and Minimum Essential Medium Eagle Alpha Modification (α-MEM, Product Number: M0644) were purchased from Sigma-Aldrich Japan K.K. Fibronectin was purchased from Biomedical Technologies Inc. Fetal calf serum was purchased from Nichirei Biosciences Inc. 2-Mercaptoethanol, penicillin-streptomycin, and TrypLE^{™} Express were purchased from Invitrogen Corp.

### Production of cell patterning culture substrate

Culture substrates each having a cell culture surface provided with a pattern of cell adhesive regions and cell non-adhesive regions were produced based on the document (Itoga K, Kobayashi J, Yamato M, Kikuchi A, Okano T., Maskless liquid-crystal-display projection photolithography for improved design flexibility of cellular micropatterns, Biomaterials, 2006, 27(15): 3005-9). A cover glass (24 mm × 50 mm, thickness: 0.12 to 0.17 mm, Matsunami Glass Ind., Ltd.) was treated with silane by an evaporation process using MPTMS. A g-line positive photoresist was spin-coated (5000 rpm, 30 sec) on the silane-treated cover glass and was prebaked at 80°C for 1 hr. A photoresist pattern where lines each having a width of 100 µm were arrayed at intervals of 300 µm was formed on the substrate surface by photolithography using a maskless exposure apparatus. The substrate was post-baked at 80°C for 1 hr. Dissolved oxygen in an aqueous solution containing 0.7 mM acrylamide and 65 µM N,N'-methylenebisacrylamide was removed by bubbling of nitrogen gas at room temperature for 20 min, and the solution was cooled to 4°C. After ammonium persulfate (2.2 mM) and TEMED (11 mM) were added to the solution, the photoresist-patterned substrate was immediately immersed in this aqueous solution. The aqueous solution was left to stand at 4°C for 3 hr with gentle stirring to fix polyacrylamide to the silanated glass surface of the substrate at the portion not coated with the photoresist. The substrate was sufficiently washed with warm water at about 40°C to remove the polyacrylamide that was not fixed to the silanated glass surface and was subsequently washed with acetone to remove the photoresist pattern of the circles on the substrate surface. The resulting substrate surface had cell adhesive circular regions made of the silanated glass surface and cell non-adhesive regions made of the polyacrylamide surface. The substrate was sufficiently washed with deionized water, dried, cut into a size of 24 mm × 20 mm, placed in a petri dish having a diameter of 35 mm, and sterilized with ethylene oxide gas. In order to enhance the cell adhesive ability before seeding, the substrate was immersed in 2 mL of D-PBS containing 30 µg/mL of fibronectin at 37°C overnight for coating the substrate surface with fibronectin.

### Cell culture

The experiment used a mouse ES cell line EMG7 (Yamashita JK, Takano M, Hiraoka-Kanie M, Shimazu C, Peishi Y, Yanagi K, et al., Prospective identification of cardiac progenitors by a novel single cell-based cardiomyocyte induction, FASEB J., 2005, 19(11): 1534-6) having an introduced gene expressing EGFP under the control of a myocardical a-inyosin heavy chain promoter. Undifferentiated ES cells were subcultured on a culture dish coated with gelatin in accordance with the culturing process described in the document. Differentiation was induced in a differentiation-inducing medium that was an α-MEM containing 5% fetal calf serum, 50 µM 2-mercaptoethanol, 5 unit/mL of penicillin, and 5 µg/mL of streptomycin. Undifferentiated ES cells collected by TrypLE^{™} Express treatment (37°C, 5 min) and suspended in the differentiation-inducing medium were seeded at 2 × 10⁴ cells/cm² on the culture substrate produced by the above-described process, and the ES cells were then cultured at 37°C in 5% carbon dioxide under saturated vapor pressure. The medium was replaced once at 3 hr after the seeding of the cells to remove the ES cells remaining on the cell non-adhesive region. Afterward, the medium was replaced once a day. The cultured cells were observed with an inverted microscope (Eclipse TE2000-U, Nikon Corporation) equipped with a HiSCA CCD camera (model C6790, Hamamatsu Photonics K.K.), and microscopic images were obtained with image analysis software AQUACOSMOS (Hamamatsu Photonics K.K.). EGFP fluorescence was observed using a dedicated optical filter (XF116-2, Omega Optical, Inc.).

### Formation of ES cell aggregate and induction of differentiation into myocardial cell on cell patterning culture substrate

The ES cells seeded on the substrate grew in the linear cell adhesive regions and reached confluence on the 3rd day of the culture. The ES cells at the boundary between the ell adhesive region and the cell non-adhesive region further grew after the cells became confluent, and these growing cells were folded onto the cell adhesive region to gradually form three-dimensional ES cell aggregates. Around the 7th day of the culture, clear EGFP fluorescence indicating differentiation into myocardial cells was observed, and some of the cell aggregates showing EGFP fluorescence started beating. Fig. 9 shows a microscopic image of cell aggregates formed on the substrate by culturing for differentiation induction for 10 days.

### INDUSTRIAL APPLICABILITY

According to the method of inducing differentiation, embryonic stem cells or artificial pluripotent stem cells such as induced pluripotent stem cells can be induced into objective cells by allowing the cells to adhere onto a substrate surface and to grow under specific conditions. The method does not require complicated procedures involved in conventional methods and can readily treat a large number of embryonic stem cells or artificial pluripotent stem cells. Thus, the present invention is significantly useful in the fields of, for example, drug discovery, pharmaceutics, medicine, and biology.

## Claims

1. A method of inducing differentiation of an embryonic stem cell or an artificial pluripotent stem cell utilizing a substrate surface having a cell non-adhesive region on the periphery of a cell adhesive region, the method comprising:
(1) allowing the cell to adhere to the cell adhesive region of the substrate surface in a differentiation-inducing medium;
(2) subsequently multiplying the cell to reach confluence on the cell adhesive surface;
(3) further continuing the culture to three-dimensionally stratify the cells from the boundary with the cell non-adhesive region surrounding the cell adhesive region; and
(4) finally binding a plurality of stratified portions formed in the cell adhesive region to produce a cell agglomerate spreading over the entire cell adhesive region with a certain thickness.

2. The method of inducing differentiation of an embryonic stem cell or an artificial pluripotent stem cell according to Claim 1, wherein the cells are stratified by continuous growth of the cells growing in the cell adhesive region after invasion onto the cell non-adhesive region and folding of a film of the cells on the cell non-adhesive region onto the cells in the cell adhesive region.

3. The method of inducing differentiation of an embryonic stem cell or an artificial pluripotent stem cell according to Claim 1 or 2, wherein the distance between at least two stratified portions formed from the boundary to the surrounding cell non-adhesive region and binding to each other in the cell adhesive region is 300 µm or less.

4. The method of inducing differentiation of an embryonic stem cell or an artificial pluripotent stem cell according to any one of Claims 1 to 3, wherein the substrate surface in the cell adhesive region is coated with an adhesive protein and/or a temperature-responsive polymer.

5. The method of inducing differentiation of an embryonic stem cell or an artificial pluripotent stem cell according to Claim 4, wherein the temperature-responsive polymer is poly(N-isopropylacrylamide).

6. The method of inducing differentiation of an embryonic stem cell or an artificial pluripotent stem cell according to Claim 4 or 5, wherein the cell agglomerate produced on the substrate surface in the cell adhesive region coated with the temperature-responsive polymer is detached only by changing the temperature of the substrate surface, with no enzyme treatment.

7. The method of inducing differentiation of an embryonic stem cell or an artificial pluripotent stem cell according to any one of Claims 1 to 6, wherein the time from seeding of the cell onto the cell adhesive region to reaching confluence is eight days or less.

8. A differentiated stem cell obtained by the method according to any one of Claims 1 to 7.

9. The differentiated stem cell according to Claim 8, wherein the cell is differentiated into a myocardial cell.

10. The differentiated stem cell according to Claim 8 or 9, the cell agglomerate containing the differentiated stem cells comprises an outermost layer having a high cell density and an inside having a low cell density.

11. The differentiated stem cell according to any one of Claims 8 to 10, wherein the cell is derived from human, dog, pig, rabbit, mouse, or rat.

12. The differentiated stem cell according to any one of Claims 8 to 11, wherein the obtained differentiate stem cell is used for transplantation therapy.

13. The differentiated stem cell according to any one of Claims 8 to 11. wherein the obtained differentiated stem cell is used for cell evaluation.
